# EUROPEAN PATENT APPLICATION

(11) **EP 4 703 001 A1**
(43) Date of publication of application: **04.03.2026**
(21) Application number: 24198206.5
(22) Date of filing: 03.09.2024
(51) Int. Cl.: A61M 27/00, A61M 39/22, A61M 39/24

(54) **IMPLANT FOR TREATMENT OF PLEURAL EFFUSION**

(71) Applicant: Ewimed GmbH, 72379 Hechingen-Boll (DE)
(72) Inventor: WIEST, Egon, 72379 Hechingen-Boll (DE); SCHEULE, Albertus, 72076 Tübingen (DE); STROBEL, Alexander, 72401 Haigerloch-Stetten (DE); ENGEL, Michael, 78050 Villingen-Schwenningen (DE)
(74) Representative: Cullinane, Marietta Bettina

(57) **Abstract**

The present invention relates to an implant for treatment of pleural effusion characterized in that it comprises a collecting chamber (4) with a proximal inlet, at least one distal lateral valve (31), which is connected to the collecting chamber (4) for draining fluid from the collecting chamber (4), and a clamping arrangement for attaching the implant (1) to tissue.

## Description

### Technical Field

The present invention relates to an implant for treating fluid build-up in a body cavity, in particular pleural effusions.

### Background of the Invention

Pathologic conditions may result in fluid build-up within a body cavity and currently require treatment through removal of the fluid from the body cavity. One such pathologic condition is pleural effusion, the build-up of fluid within the pleural cavity, and particularly fluid build-up in the space between the visceral pleural layer and the parietal pleural layer. Pleural effusion is typically treated by draining excess fluid from the pleural cavity using a catheter connected to an external suction source (e.g., a pump) or using gravity. However, such an approach is disadvantageous in that fluid often builds up again in the plural cavity, which then requires another catheter-based intervention to be performed for draining, which may cause infections (e.g., empyema). In addition, the fluid will not be transfused back into the patient and excessive loss may cause hypoproteinemia.

### Description of the Invention

Starting from the known state of the art, the problem to be solved is thus to provide a device for the treatment of fluid build-up in a body cavity, particularly fluid build-up in the pleural cavity, which can ensure reliable extraction of the fluid in a simple manner. In addition, the device is to eliminate the problems encountered when using drainage catheters, allow for the recirculation of fluid removed from the body cavity without removing it from the patient's body, and to place as little burden on the patient as possible.

The problem is solved by an implant with the features according to claim 1. Preferred embodiments are disclosed in the dependent claims and are described in the following description.

The present invention relates to an implant for treatment of pleural effusion. The implant is characterized in that it comprises a collecting chamber with a proximal inlet, at least one distal lateral valve, which is connected to the collecting chamber for draining fluid from the collecting chamber, and a clamping arrangement for attaching the implant to tissue.

The implant according to the invention can also be referred to as a valve implant or pleural valve implant.

The implant comprises a collecting chamber, at least one distal lateral valve and a clamping arrangement. The collecting chamber has a proximal inlet. The collecting chamber may have a cylindrical shape. The proximal inlet may be the proximal rim of the collecting chamber.

If not specified otherwise, the term distal designates the side of the implant or its elements which is or are located within the abdomen after implanting. The term proximal designates the side of the implant or its elements which is or are located within the pleural cavity after implanting. Preferably, the implant is implanted into an incision in the diaphragm which separates the abdominal cavity from the pleural cavity. In a preferred embodiment, the implant is implanted from the direction of the pleural cavity through the incision into the abdominal cavity. The direction distal can therefore also designate the direction from the pleural to the abdominal cavity and the direction proximal designates the opposite direction. If not specified otherwise, the term downwards designates a movement in the distal direction or towards a distal end. The term bottom preferably refers to the distal side of a component or element. If not specified otherwise, the term upwards designates a movement in the proximal direction or towards a proximal end. The term upper side preferably refers to the proximal side of a component or element.

If not specified otherwise, the shape of the implant, which it has after manufacturing, will be referred to as original shape. The shape of the implant which it assumes after implantation manufacturing, will be referred to as final shape. The original shape and the final shape are preferably the same or only differ in the distance of components of the clamping arrangement towards each other, since in the final shape, the tissue will be clamped between the components of the clamping arrangement. The shape of the implant during delivery of the implant to the implant site will be referred to as delivery shape.

If not specified otherwise, the relative position of the elements of the implant towards each other is the position of elements in the final shape of the implant.

The collecting chamber can also be referred to as collection area. The collecting chamber defines a space in the implant, where liquid which enters the implant via the proximal inlet, is received and preferably collected before reaching the at least one valve of the implant. In one embodiment, the collecting chamber is a cylindrical chamber having an open end at the proximal side, which forms the proximal inlet, and being closed at the distal side. In the lateral direction, the collecting chamber has at least one opening connecting to the at least one lateral valve. The at least one opening is preferably located at the distal end of the wall defining the cylindrical chamber. The opening can for example be a slit extending from the distal end of the wall defining the cylindrical chamber in a proximal direction. In one embodiment, the collecting chamber is formed by a central ring of a core body and portions of a cover, which covers at least part of the core body. In one embodiment, the collecting chamber therefore is formed by the central ring, the portion of the cover surrounding the central ring and extending in distal direction over the distal end of the central ring as well as a central portion of a lower portion of the cover, which closes the distal side of the collecting chamber.

The implant further comprises at least one distal lateral valve. A distal lateral valve is a valve which is provided at the distal end of the collecting chamber or preferably adjacent to the distal end of the collecting chamber at the outer surface of the collecting chamber and extends in a radially outward direction from the outer surface of the collecting chamber. After implanting the implant, the valve is or the valves are positioned distal to the tissue, in particular diaphragm, i.e. in the abdominal cavity, while the wall of the collecting chamber extends through the diaphragm in a proximal direction. The respective openings of the collecting chamber in this case extend over the distance between the distal end of the collecting chamber and the distal surface of the tissue to which the implant is to be fastened. Each distal lateral valve is connected to the collecting chamber. The distal lateral valve(s) are connected to the collecting chamber such that fluid can flow from the collecting chamber into the respective valve(s). The fluid can then leave the implant through the valve(s). The distal lateral valve(s) may be attached to the collecting chamber. In a preferred embodiment, the at least one distal lateral valve is, however, integrally formed with the collecting chamber.

A lateral valve is a valve, where at least one outlet opening is arranged such that fluid from the valve leaves the valve in a direction inclined to the collecting chamber, in particular inclined to the wall of the collecting chamber. Preferably, the lateral valve(s) extend perpendicular from the outer wall of the collecting chamber. In one embodiment, the valves are provided such that an angle in the range of 1 ° to 90°, in particular in the range of 5° to 90°, is enclosed between an imaginary distal elongation of the outer wall of the collecting chamber and the valve, i.e. the valve(s) are at an angle which is between the perpendicular direction to the outer wall and the imaginary distal elongation.

The distal lateral valve(s) are hereinafter simply referred to as valve(s). The at least one distal lateral valve is referred to as distal lateral valve(s) or simply as valve(s). The release of the fluid occurs once the pressure at the proximal side, in particular in the pleural cavity, is larger than the pressure at the distal side of the implant, in particular in the abdominal cavity.

In one embodiment, the valve comprises a cover with a circular bottom portion and a funnel-shaped upper portion. In this embodiment, the outlet opening of the valve extends over the entire circumference of the cover, i.e. is formed by the gap between the outer circumference of the circular bottom portion and the largest circumference of the upper portion. In this case only one valve is provided at the implant. It is, however, also within the scope of the invention, that the single valve is provided over only part of the circumference of the implant. In this embodiment, the valve may extend from the collecting chamber in only one radial direction.

In an alternative embodiment, the implant comprises more than one valve. In that case the valves can be arranged in circumferential distance to each other.

The implant further comprises a clamping arrangement. The clamping arrangement serves for attaching the implant to tissue. Preferably, the clamping arrangement serves for attaching the implant at an incision of a tissue by clamping at least part of the tissue surrounding the incision with the clamping arrangement.

By providing at least one lateral distal valve at a collecting chamber of an implant, the fluid can be released from the implant. Preferably the fluid can be released in different radial directions from the collecting chamber. Thereby, even if part of one valve or one of several valves is blocked, the fluid may still exit the implant, in particular by a different part of the one valve or at least one other valve of several valves. In addition, since the valve(s) are lateral valves, the implant can be used at a site, where organs are close to the tissue at which the implant is to be attached, in particular the diaphragm. Such organ may for example be the liver. Blocking of release of fluid by the organ, which would occur with a valve directed parallel to the extension of the collecting chamber, can be avoided by providing one or more lateral valves. Thereby, a reliable release of fluid can be achieved.

In a preferred embodiment, the implant comprises at least two distal lateral valves. These valves preferably extend in different radial directions from the collecting chamber. The valves can be arranged at intervals over the circumference of the collecting chambers. By providing at least two valves a potential clogging or blocking of one of the valves can be compensated by the at least one other of the valves. As the at least two valves preferably extend in different directions, in particular different radial directions from the collecting chamber, a potential blocking of one or more of the valves by an organ can reliably be compensated by at least one other of the valves.

The implant can be made of two parts. The two parts can be connected to each other to form the implant. The assembly of the implant may be performed before implantation. In an alternative embodiment, the assembly of the implant, namely the connection of the two parts, can be performed during implantation. In this case, the two parts may be connected to each other by snapping one part to the other, for example by clips.

One part of the implant may form the distal part of the implant, in particular the distal part of the collecting chamber, the valves and distal parts of the clamping arrangement, in particular distal arm(s). The other part of the implant may form the proximal part of the implant, in particular the proximal part of the collecting chamber and the proximal part of the clamping arrangement, in particular proximal arm(s).

In an alternative embodiment, one part of the implant may form the clamping arrangement, in particular a central ring with proximal arm(s) and distal arm(s) and the other part may form an outer part of the collection chamber and the valves.

According to a preferred embodiment, the implant comprises a core body and a cover. The core body can be made of plastic material or of metal. In one embodiment the core body is made of a polymer, such as PTFE (polytetrafluoroethylene) or silicone or a thermoplastic elastomer (TPE) or rubber. In another embodiment the core body is made of metal and the cover is made of plastic material, in particular polymer, such as PTFE (polytetrafluorethylene) or silicone. The core body can be made of a metal, which has superelastic properties. The metal can be a shape memory alloy. In a preferred embodiment, the core body is made of Nitinol.

One advantage of providing a core body and a cover is that the core body can provide strength and preferably flexibility, while the cover can provide protection of the surrounding tissue, prevent overgrowing and can provide sealing of the incision around the implant in a simple manner.

The core body preferably comprising a central ring having a distal and a proximal end. The core body can also comprise parts of or form the entire clamping arrangement, In particular the core body can comprise or consist of the central ring, the distal arm(s) and proximal arm(s). These arms can be arranged at the distal end and proximal end of the central ring, respectively. The wall of the central ring extends between the distal and the proximal end thereof. The cross-section of the central ring may be circular or oval. The wall thickness of the core body and in particular of the wall of the central ring may be, for example, in the range of 0.15 mm to 0.5 mm, e.g. 0.29 mm. The outer diameter of the central ring may be, for example, in the range from 5mm to 7mm. The height or length of the central ring, i.e. the distance between the proximal and distal end may be, for example, in the range from 1.5mm to 4mm, e.g. 2.5mm.

The central ring imparts stability to the implant. In particular, the central ring stabilizes the collecting chamber and can prevent collapsing of the collecting chamber during use. In addition, the central ring can serve as a support of the other clamping arrangement elements, in particular the proximal and distal arm(s).

The cover is a part of the implant which at least comprises the at least one valve and covers at least part of the core body. In addition, the cover preferably forms a part of the collecting chamber, in particular the bottom or distal side of the collecting chamber.

The cover can cover all surfaces of the core body. The cover may be in direct contact to the respective surface or may at least partially be provided with a distance to the surface, while still protecting the surface from the surroundings. In one embodiment, the cover only covers some surfaces of the core body. For example, the cover can only cover the distal arm(s) of the clamping arrangement, in particular the struts. In addition, the cover may also cover the central ring. The proximal arms, particular the clamps, may be with or without cover.

According to one embodiment, the cover comprises the at least one valve of the implant. This means that the valve(s) of the implant can be formed by the cover. The cover preferably further comprises a collecting portion and a central bottom portion. The collecting portion preferably at least surrounds part of the central ring and preferably extends in a distal direction beyond the distal end of the central ring. The collecting portion preferably has a cylindrical shape. At the distal end of the collecting portion of the cover a transitional area may be provided, which leads to a flange extending outwardly. The flange may comprise at least a top layer of the valve(s). The central bottom portion of the cover is preferably distally spaced from the distal end of the central ring. The central bottom portion is preferably part of a bottom portion or layer of the cover, which can include further sections, such as a bottom layer of the valve(s).

Preferably, the collecting portion and the central bottom portion form the outer limitations of the collecting chamber of the implant. In one embodiment, where the implant comprises a core body with a central ring, at least the central ring can be positioned within the collecting portion of the cover and can thereby form the inner part of the surrounding wall of the collecting chamber.

According to a one embodiment, the cover can thus consist of an upper portion and a bottom portion, where the upper portion comprises the collecting portion and a top layer of the valve(s), while the bottom portion comprises the central bottom portion and the bottom layer of the valve(s).

By proving the valve(s) as part of a cover which forms at least part of the collecting chamber, the manufacturing of the implant can be simplified. In addition, the flow path for fluid from the collecting chamber to the valve(s) can be provided in a simple manner.

The clamping arrangement can consist of one proximal arm, one distal arm and a connecting member such as a central ring of the core body. In this case, the proximal arm and distal arm are preferably arrange at the same angular position of the central ring, which means they extend outwards from the central ring in the radial direction and are parallel to each other. Thereby the tissue can be clamped between the radial and distal arm.

Preferably, the clamping arrangement comprises at least two proximal arms and at least two distal arms. The proximal arms at least partially extend radially outwards from the central ring. The proximal arms are preferably provided at the proximal end of the central ring. The proximal arms will hereinafter also be referred to as clamps. The distal arms at least partially extend radially outwards from the central ring. The distal arms are preferably provided at the distal end of the central ring. The distal arms will hereinafter also be referred to as struts.

The radially outwards extending arms may extend perpendicular to the direction of the collecting chamber, i.e. perpendicular to the proximal-to-distal direction, or may be inclined in the distal or proximal direction.

As the arms at least partially extend radially outwards, the tissue, at which the implant is to be positioned, can be clamped between the distal and the proximal arms. By using more than one distal arm and more than one proximal arm, the fixation of the implant to the tissue can be improved.

By providing proximal arms and distal arms at the central ring of the core body, the axial distance between the distal and proximal arms can be set during manufacturing of the core body and thus a good grip of the arms on the tissue to be received between these arms can be achieved upon implantation.

According to a preferred embodiment, the proximal arms partially extend over the proximal end of the central ring in proximal direction. This extension can be referred to as the proximally oriented section of the proximal arms. The proximal rim of the central rim forms the proximal inlet of the collecting chamber. The proximally oriented section of the proximal arms over the proximal end of the central ring can prevent that the proximal side of the collecting chamber, in particular the proximal rim of the central ring, which forms the inlet, is blocked, when the implant is used. Such blocking can for example be caused by the lung. As the proximal arms extend at least partially in the proximal direction, they form a cage, which keeps the lung at a distance to the proximal end of the central ring and thus allows fluid to enter at least through the circumferential distance between adjacent proximal arms into the collecting chamber.

According to one embodiment, the distal arms are offset to the proximal arms in a circumferential direction of the central ring. This means, that the distal arms are arranged at different angular positions of the central ring than the proximal arms. By this arrangement squeezing and thereby potentially harming the tissue between a strut and a clamp can be avoided.

Preferably the core body is formed as a single component. In this embodiment, the central ring, the clamps and the struts are all integral parts of the core body. The core body can, for example, be made from a metal or plastic tube. The clamps and struts can be formed by providing longitudinal slits into the tube at both ends, thereby leaving the central ring. After the slits have been made, for example by laser cutting, the clamps and struts can be bent relative to the central ring. Thereby, the original shape of the core body in the implant, i.e. the shape which the core body in the implant has after manufacturing, can be produced. In an alternative embodiment, the core body can be manufactured by 3-D printing. In this embodiment the central ring and the arms as well as the original shape of the core body of the implant can be manufactured in one single manufacturing step, thereby further simplifying the manufacturing of the implant.

Forming the central ring and the arms as a single component has several advantages. In particular, the manufacturing is simplified, the relative position of the arms to the central ring and of the arms to each other can be set. Finally, breaking-off of the arms from the central ring is less likely than in case of an attachment of the arms to the central ring, when the arms are flexed relative to the central ring. Such flexing or bending will in particular be necessary for inserting the implant into the patient's body and implanting at the implanting site.

According to one embodiment, the distal arms comprise a bending section, where the distal arms are bent from the distal direction into a radially outward direction. Preferably, the bending section extends distally over the distal end of the central ring. In this embodiment, each strut preferably consists of a distally oriented section, a bending section and a radial section. The proximal end of the distally oriented section is located at the distal end of the central ring and the distal end of the distally oriented section transitions into the bending section from which a radial section extends in a radial direction. Thereby, also if the distal arms are formed by struts, which at least with their radial sections extend perpendicular from the wall of the central ring, a distance is provided between the distal end of the central ring and the radial section of the strut, in particular the distal side of the struts in the radial section. In this distance the slots, which are formed by the circumferential distance between neighboring distal arms, in particular the distance between distally orientated sections of neighboring distal arms, allow fluid to reach the inlet opening of a valve provided at the wall of the collecting chamber.

According to one embodiment, the proximal arms at least partially extend into a radially outward direction and comprise a bending section, where the proximal arms are bent from the proximal direction to a direction between a perpendicular direction from the central ring and the distal direction. The proximal arms are thus preferably bent by an angle of more than 90° from the proximal direction. The proximal arms thus have a downward slanted section adjacent to the bending section. The proximal arms thus preferably have a proximally oriented section, a bending section and a slanted section. The radial end of the slanted section may be referred to as an ending section and may be at a different angle than the slanted section. In a preferred embodiment the radial outer ends of the proximal arms lie in the level of the distal arms. The provision of a bending section and the downward slanted section, ensures on one hand, that the distal arm can reach the tissue. On the other hand, it prevents harming an organ, in particular the lung, which might come into contact with the proximal arms.

In particular, when the distal arms, i.e. struts are formed as struts extending radially perpendicular to the central ring and the radial outer ends of the proximal arms lie in the level of the distal arms, it can be ensure, that the struts are pulled towards the tissue, if the clamps are in contact with opposite side of the tissue. If the tissue is the diaphragm, once the implant is implanted, the struts can rest against the abdominal side of the diaphragm and the clamps can rest against the side of the diaphragm facing the pleural cavity.

According to one embodiment, the clamps and/or struts each have a hole near their radial outer end. By providing such holes, the implant with the core body comprising clamps and/or struts can be pulled by string or wire during implanting process, where necessary. In particular, the core body can be pulled via the strings or wires attached to the respective holes in the clamps and/or struts in the proximal direction. In this case, once the struts passed the incision, the pulling stings or wires can be loosened or cut, so that the struts can expand to the radially outward direction. Once the struts are expanded, the implant can be pulled by the strings or wires at the clamps in the proximal direction, until the struts rests against the distal side of the tissue. At that point the pulling strings or wires pulling the clamps in the proximal direction can be loosened or cut. Thereby the clamps can reassume their radially outwards directed position and can thus clamp the tissue together with the struts.

In an alternative embodiment, no holes and no pulling strings or wires are provided. In this case, the implant can be delivered to the incision site by a sheath, which can also be referred to as delivery pipe. The implant is inserted into the sheath in a shape, where the struts and clamps are aligned with the outer circumference of the central ring of the core body of the implant. The shape that the implant assumes in the sheath is referred to as the delivery shape. In the delivery shape, the struts extend in the distal direction from the central ring and the clamps extend in the proximal direction from the central ring. Once the implant is released from the sheath, the implant assumes its final shape, which means it tries to regain its original shape, where the struts and clamps extend in radial direction from the central ring. Since the tissue is clamped between the distal and proximal arms, the distance between the outer ends a distal arm and a corresponding proximal arm will be larger than in the original shape of the implant. It is thus possible, that the implant will not reach its original shape but a final shape, which only differs from the original shape in the distance between distal and proximal arm. If the tissue is soft enough or if the distal and proximal arms are offset to each other at a large circumferential distance, the original shape and final shape of the implant may be the same. The reshaping of the implant from the delivery shape to the final shape can be due to the superelasticity of the material of which the implant is made.

The number of struts and can be equal to the number of clamps. According to one embodiment, the core body comprises three struts and three clamps, preferably five struts and five clamps. By providing at least three struts and at least three clamps, undesired turning of the implant in the incision can be avoided. At the same time with the embodiment with three struts and three clamps, the distance between the adjacent struts and the distance between adjacent clamps can be increased. Finally, by providing at least three struts an improved sealing of the incision by the cover can be achieved.

In an alternative embodiment the number of struts differs from the number of clamps. In particular, the number of clamps can be higher than the number of struts. For example, the number of clamps can be eight, while the number of struts can be five or less. Alternatively, the number of struts, for example, can be higher than the number of clamps. For example, the number of struts can be eight, while the number of clamps can be five or less.

The number of struts and/or clamps is not limited to the given examples.

According to a preferred embodiment, the implant comprises at least one valve which extends from the collecting chamber in a radially outwards direction and each valve comprises an outlet opening at its radial outer end. In a further preferred embodiment each valve has at least one additional outlet opening at the distal side of the valve. The additional outlet opening is provided at a distance from the radially outermost end of the valve. The additional outlet opening(s) ensure that fluid can exit the valve, even if the outlet opening at the radial outer end is blocked. The pressure difference required for activating, i.e. opening the valve can be changed by changing the width and/or shape of the valve. In one embodiment the valve has a funnel shape, which may increase or decrease towards the outermost end of the valve.

According to one embodiment, the valves are formed in the cover between adjacent struts of the core body. With this embodiment, the struts can pull the valves into their position, in particular into a radially outwards direction from the collecting chamber, when the struts are expanded during implanting of the implant. For this purpose, the valves are preferably at least along a part of their length connected to the portion of the cover that covers the adjacent struts. The connection may be at the inner radial portion of the valves, i.e. close to the collecting chamber, or at the outer radial portion of the valves, i.e. at or close to their radial end.

In a preferred embodiment, each valve is formed by two plastic layers. The sides of the valve in this case may be formed by sealing lines sealing the two plastic layers together. The two layers may be formed by a distal layer, which may be part of a lower portion of the cover, which also covers the distal side of the struts and a proximal layer, which may be part of an upper portion of the cover, which also covers the proximal side of the strut. The proximal layer may be a flange formed at the distal end of the collecting portion of the cover. The distal layer is preferably the bottom of the cover, which also includes the central portion, which forms the bottom of the collecting chamber.

One advantage of a valve made of two layers is the small thickness of the valve, which for example allows the use of the implant also at a location, where the liver is close to the diaphragm.

The two-layer valve can serve as a one-way valve, since the areal pressure in the abdominal cavity causes closing of the valve. Only if the pressure difference between the pleural cavity and the abdominal cavity reaches a prescribed value, the valve will open. The length and width of the valve has an influence on the activation pressure.

According to a preferred embodiment, the cover comprises a central portion forming a bottom the collecting chamber. The central portion can be one piece with a distal layer, which can also be referred to as a bottom layer, of the at least the one valve made of two layers. In addition, a distal coating of the struts may be part of the distal portion of the cover. The distal portion of the cover may have a disc shape or may have a star shape, wherein stripes of the cover material extending from the central portion are aligned with the struts and a proximal layer of the valve(s) made of two layers.

In this case, the cover may comprise a proximal portion which comprises the collecting portion and the proximal layer, which can also be referred to as top layer, of the at least one valve made of two layers. In addition a proximal coating of the struts may be included in the proximal portion.

Alternatively to a cover made of a proximal and distal layer or portion, the cover may be made as an integral element.

According to one embodiment, the cover comprises portions which individually enclose each of the struts and the clamps.

### Brief description of the Figures

Some embodiments of the invention are described in more detail with reference to the enclosed figures, wherein:
Figure 1 shows a perspective view of an embodiment of the implant according to the invention
Figure 2 shows a schematic sectional view of the core body of an embodiment of an implant according to the invention;
Figures 3a and 3b show a perspective view of an embodiment of the core body and of an implant according to the invention;
Figures 4a and 4b show a perspective view of an embodiment of the core body and of an implant according to the invention;
Figures 5a and 5b show a top view and a bottom view of the cover of an embodiment of an implant according to the invention;
Figures 6a and 6b show a top view and a bottom view of the cover of another embodiment of an implant according to the invention;
Figures 7a and 7b show a top view and a bottom view of the cover of another embodiment of an implant according to the invention;
Figures 8a and 8b show a schematic sectional view and bottom view of a valve of an embodiment of an implant according to the invention;
Figure 9 shows a schematic view of an embodiment of the implant in a sheath; and
Figures 10a and 10b show schematic cross-sectional views of different embodiments of the valves of the implant.

### Detailed Description of preferred Embodiments

Preferred embodiments are described hereinafter with reference to the figures. Therein, same or similar elements in different figures have the same reference numbers and - where appropriate - will only be described once to avoid redundancies.

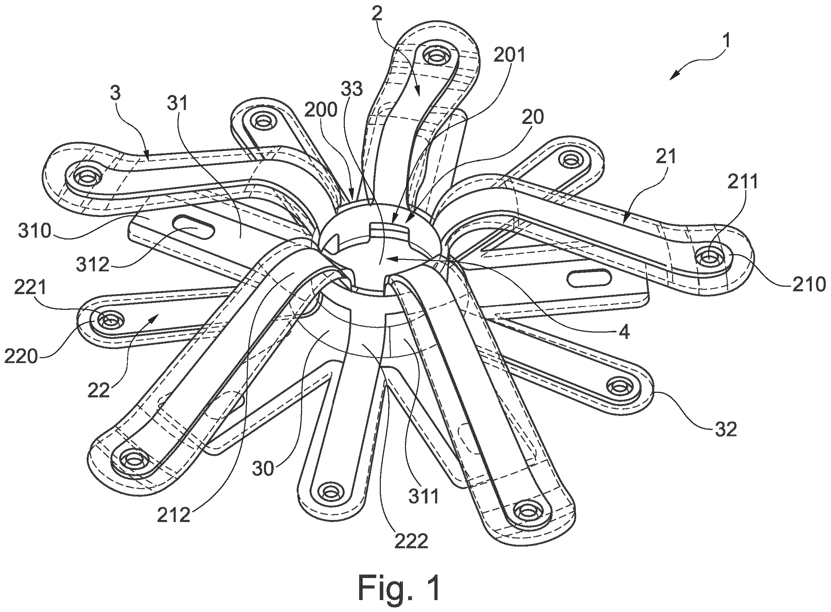

In Figure 1 a perspective view of an embodiment of the implant 1 for treatment of pleural effusion according to the invention is shown. The implant 1 is shown in its original shape.

The implant comprises a collecting chamber 4, distal lateral valves, which will hereinafter be referred to as valves 31, and a clamping arrangement comprising proximal arms, which will hereinafter be referred to as clamps 21, and distal arms, which will hereinafter be referred to as struts 22. In the depicted embodiment, the implant 1 has five clamps 21 and five struts 22. Figure 1 shows a perspective top view. The upper part pf the implant 1 therein is the proximal part and the bottom part is the distal part. The collecting chamber 4 has a proximal inlet, which is located in the center of the implant 1. The valves 31 are formed at the distal side of the implant 1, are connected to the collecting chamber 4 and extend laterally from the collecting chamber 4.

The implant 1 can be positioned at a tissue (not shown), in particular the diaphragm of the patient. Once implanted, the clamps 21 and the proximal inlet of the collecting chamber 4 will be located in the pleural cavity, while the struts 22 and the valves 31 will be located in the abdominal cavity. The collecting chamber 4 extends through the tissue.

In the embodiment shown in Figure 1 the implant 1 consists of a core body 2 and a cover 3.

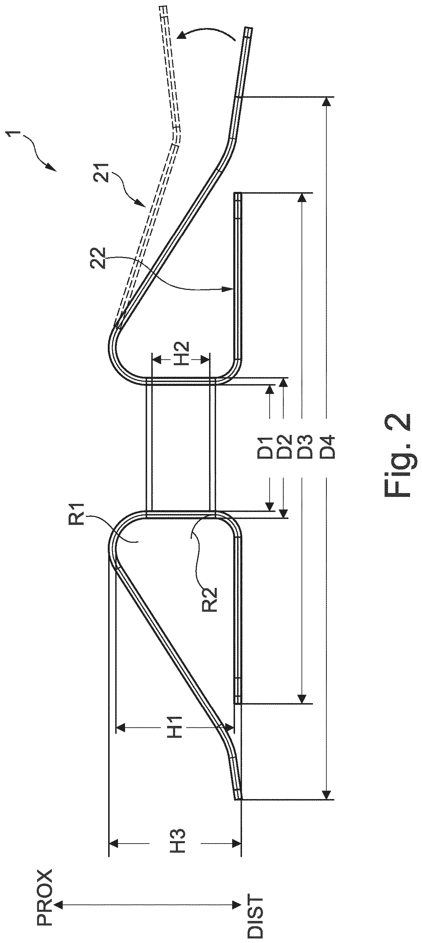

The core body 2 will now be described in more detail with reference to Figure 1 and 2. The directions proximal (PROX) and distal (DIST) are schematically indicated in Figure 2. The core body 2 has a central ring 20. The proximal end 200 of the central ring 20 forms the inlet of the collecting chamber 4. At the proximal end 200 of the central ring 20 the clamps 21 are provided. The struts 22 are provided at the distal end 201 of the central ring 20.

In the depicted embodiment, the clamps 21 extend from the central ring 20 in the proximal direction to then bend in a bending section (212) radially outwards and to subsequently extend in distal direction in a slanted manner so as to form a slanted section of the clamp 21. The clamps 21 in the depicted embodiment further include an ending section which is arranged at an angle to the slanting section.

In the depicted embodiment, the struts 22 extend from the central ring 20 in the distal direction to then bend radially outwards via a bending section 222. The depicted struts 22 do not have a slanted portion but after the bending section 222 extend perpendicular from the outer circumference of the central ring 20.

In the original shape of the implant 1, the radial outer end of the clamps 21 lies in the level of the struts 22 or even extends distally over this level. This is schematically shown in Figure 2 on the right hand side. Also shown in Figure 2 on the right hand side is a movement that the clamps 21 can perform. In particular, the outer end of the clamps 21 can be moved in the proximal direction. This movement will be described in more detail later with respect to delivery of the implant 1 to the incision site. In addition, the flexibility of the arms, in particular the clamps 21 as indicated in Figure 2 show, how the tissue can be clamped between the clamps 21 and struts 22. If the clamps 21 are flexed in the proximal direction as shown in Figure 2 on the right hand side, the tissue will be clamped between the transition from the slanted to the radial section of the clamps 21 and the radial section of the struts 22.

In other embodiments of the core body 2 (not shown), the struts 22 and in particular the radial section of the struts 22 do not extend perpendicular from the outer circumference of the central ring 20 and thus the wall of the collecting chamber 4, but extend at an angle α between an imaginary distal elongation (IDE) of the wall of the collecting chamber 4 and the distal side of the radial section of the struts 22 of less than 90°.

In the embodiment depicted in Figure 1, the clamps 21 and struts 22 both have a hole 211, 221 close to their radial outer end. A string or wire (not shown) can be led through the hole 211, 221 of the clamp 21 or strut 22, thereby allowing to apply pulling force in the proximal direction.

The struts 22 are offset to the clamps 21 in a circumferential direction of the central ring 20.

The dimensions of one embodiment of the core body 2 are indicated in Figure 2. The bending radius R1 by which the clamps 21 are bent outwardly can for example be in the range from 1 mm to 8 mm. The bending radius R1 is larger than bending radius R2, by which the struts 22 are bent outwardly in the bending section 222. The bending radius R2 can for example be in the range from 0.5 mm to 8 mm. The inner diameter D1 of the central ring 20 can for example be in the range from 4 mm to 9 mm. The outer diameter D2 of the central ring 20 can for example be in the range from 4.3 mm to 12 mm. The diameter D3 of the core body 2 at the struts 22 can for example be in the range from 8 mm to 20 mm. The diameter D4 of the core body 2 at the clamps can for example be in the range from 10 mm to 40 mm. The height H3 of the implant can for example be in the range from 6 mm to 24 mm. The height H2 of the central ring 20 can for example be in the range from 2 mm to 8 mm. The maximal height H1 of the core body for receiving tissue can for example be in the range from 0,3 mm to 3mm.

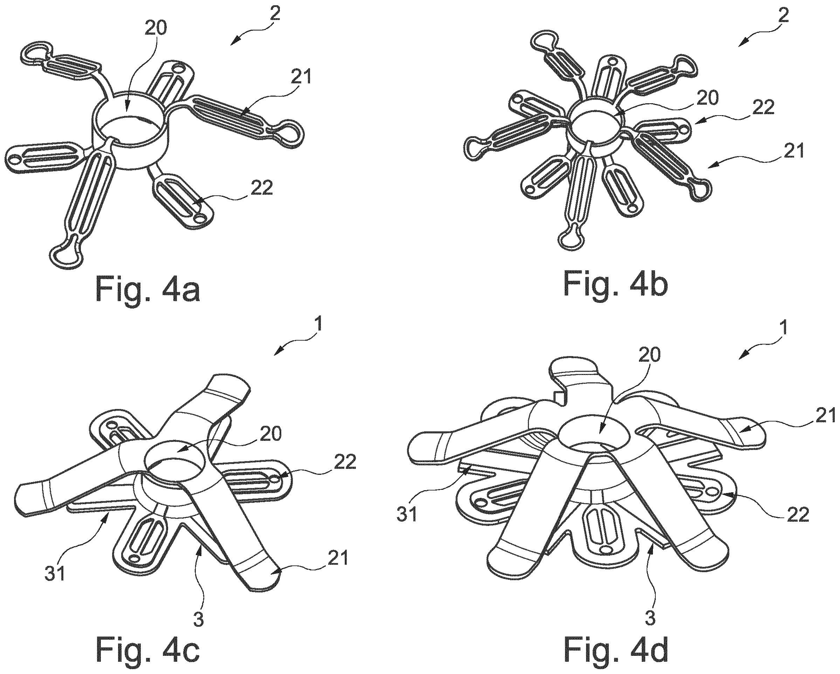

Further embodiments of core bodies 2 are shown in Figures 3a and 4a. The embodiments shown in Figure 3a and 4a differs from the embodiment shown in Figures 1 and 2 in the number of clamps 21 and struts 22 and in that the clamps 21 and struts 22 of Figures 3a and 4a have a skeleton structure. In particular, the width of the clamps 21 and struts 22 varies over their lengths. In addition, slots extending over the wider parts of the clamps 21 and struts 22 in their length direction are provided.

The embodiment of the core body 2 shown in Figure 3a only differs from the embodiment shown in Figure 4a in that it has three clamps 21 and three struts 22. Thereby, the circumferential distance between neighboring clamps 21 of the embodiment of Figure 3a is larger than in the embodiment of Figure 4a. Also, the distance between neighboring struts 22 of the embodiment of Figure 3a is larger than in the embodiment of Figure 4a.

The implants 1 in Figures 3b and 4b are formed from the core bodies 2 of Figure 3a and 4a, respectively. The core body 2 is coated with a cover 3. In the depicted embodiment, the cover 3 covers the outer wall of the central ring 20 as well as the clamps 21 and struts 22. In addition, the cover 3 forms the valves 31 of the implant 1 and the central bottom portion 33. The valves 31 are arranged in the circumferential direction between two struts 22.

One embodiment of the cover 3 of the implant 1 will now be described in more detail with reference to Figure 1. The cover 3 may also be referred to as coating or laminate.

In the embodiment shown in Figure 1, the cover 3 is provided on the surfaces of the core body 2. In particular, each clamp 21 and each strut 22 is coated with the cover 3. In addition, the cover 3 also covers the outer circumference of the central ring 20. In Figure 1 the inner circumference of the central ring 20 is not covered by the cover 3. It is, however within the scope of the present invention to also provide the cover on the inner circumference of the central ring 20. It is also within the scope of the invention that only the struts 22 are coated with the cover 3, while the clamps 21 are not coated.

The depicted cover 3 also has a bottom portion, which is provided at the distal side of the implant 1 and can therefore also be referred to as distal layer or bottom layer of the cover 3. The bottom layer of the cover 3 comprises a central portion 33, which is located distal to the distal end of the central ring 20. From the central portion 33 cover sections for the distal side of the struts 22 and the bottom side of the valves 31 extend in radial direction.

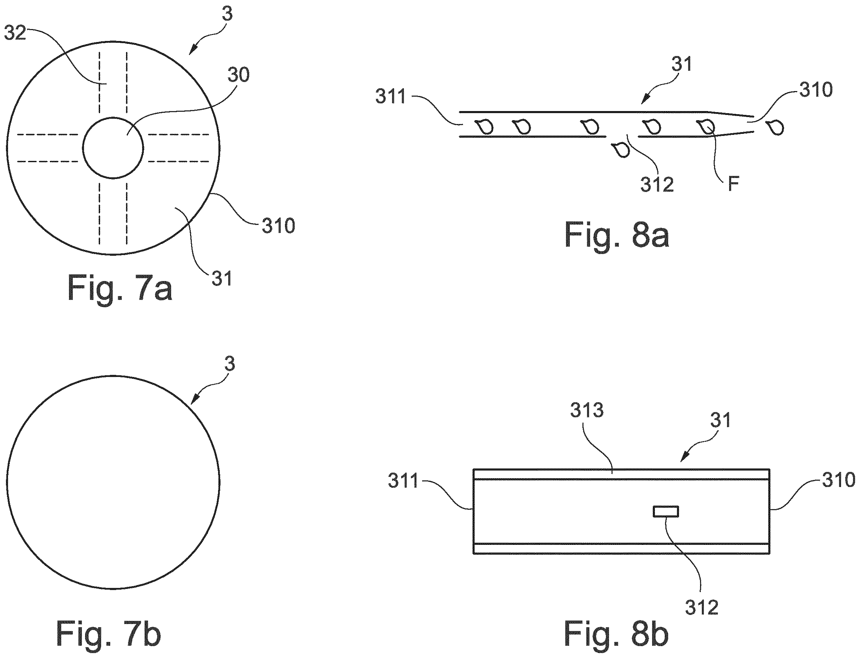

In the depicted embodiments, the valves 31 are formed as a two-layer valve, which functions as a check valve. In particular the valve 31 consists of a distal layer and a proximal layer. At the outer radial end of the valve 31 an outlet opening 310 is provided. At the side of the valve 31 facing towards the collecting chamber 4, the inlet opening 311 is formed. As can be derived from Figure 1 and 8, the valve 31 of the depicted embodiment has an additional outlet opening 312 which is provided in the distal side of the valve 31, in particular in the distal layer of the valve 31. According to one embodiment and as schematically shown in Figure 8b the two layers of the valve 31 can be sealed to one another along the length of the valve 31 by a sealing line 313. It is, however, also possible that the two layers of the valve 31 are provided by using a flattened tube. In addition, it is possible to provide support elements (not shown) in addition to the sealing line 313 to the valve(s) 31 to keep the valve(s) 31 in a predetermined direction in the final shape of the implant 1.

The valves 31 are provided between two adjacent struts 22. In this way, each valve 31 can be attached over at least part of its length to the cover section 32 for the strut 22. Thereby, a sealing in the area of the incision in addition to the sealing provided by the central portion 33 can be achieved. In addition, the attachment of the valves 31 over at least part of its length to the cover section 32 for the strut 22 allows the struts 22 to apply a certain force onto the valve(s) 31. In particular, when the struts 22 assume their final position, which may be perpendicular to the wall of the collecting chamber 4, also the valves 31 will be forced in this direction. The struts 22 can thus function as support of the valve(s) 31.

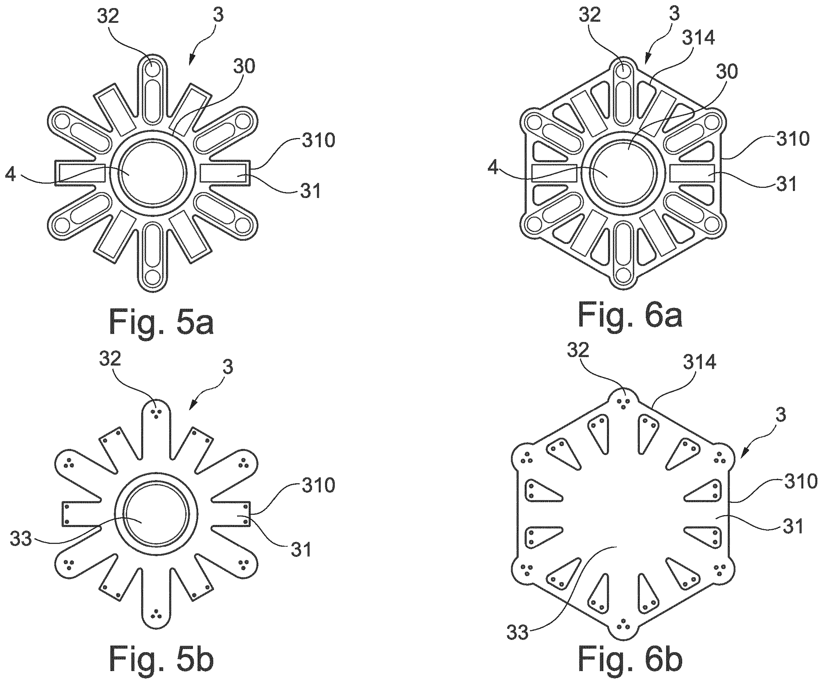

Figures 5 and 6 show two different embodiments of the cover 3 in the area of the central ring and the struts (not shown in Figures 5 and 6). In both embodiments the central portion 33 of the bottom of the cover 3 and the distal sides of the cover sections 32 for the strut 22 and of the valves 31 are part of one layer.

Figure 5 shows the cover 3 as used in embodiments of the implant 1 as shown in Figures 1 and 4, where the outer ends of the cover sections 32 for the strut 22 and of the valves 31 are separated from each other. In the embodiment of the cover 3 shown in Figure 6 connecting stripes 314 of cover material are provided between the outer radial ends of each cover sections 32 for the strut 22 and of the adjacent valve 31 and vice versa. These connecting stripes 31 further ensure that the position of the valve(s) 31 in the final shape of the implant 1 correspond to the position that the struts 22 have in that shape.

In an alternative embodiment shown in Figures 7a and 7b, the cover 3 has a circular shape. In that embodiment the valve 31 are formed by the section of the circular shape between two adjacent cover sections 32 for the struts 22. In the depicted embodiment four valves 31 are provided. The width of the valves 31 increases from the collecting portion 30 to the radially outward outlet opening 310. By the increased length of the outlet opening 310 along the outer circumference of the circular cover 3, the initial pressure for opening the valve 31 is decreased.

In one embodiment the cover 3 of Figure 7a and 7b consist of a distal disk-shaped portion and a proximal portion, which is formed by the collecting portion 30 and a circular flange surrounding collecting portion 30. In this embodiment, the circular flange may be connected to the proximal side of the struts 22 of the core body 2 of the implant 1. The distal disk-shaped portion is connected to the distal part to the collecting portion 30 in such a way, that fluid can exit from the collecting portion 30 and reach the outer circumference of the distal disk-shaped portion and thereby leave the implant. The outlet opening of the valve 31 in this case is formed by the entire outer circumference of the distal disk-shaped portion of the cover 3.

The collecting chamber 4 of the implant 1 preferably comprises the collecting portion 30 of the cover 3. The collecting portion 30 is formed by the portion of the cover 3 surrounding the central ring 20 as well as the portion extending from the distal end of the central ring 20 towards the bottom layer of the cover 3. The inlet of the valve 31 is formed between this extending part of the collecting portion 30 and the bottom layer of the cover 3. In particular, the inlet of the valve 31 is located in a gap provided between the bending sections 222 of two adjacent struts 22.

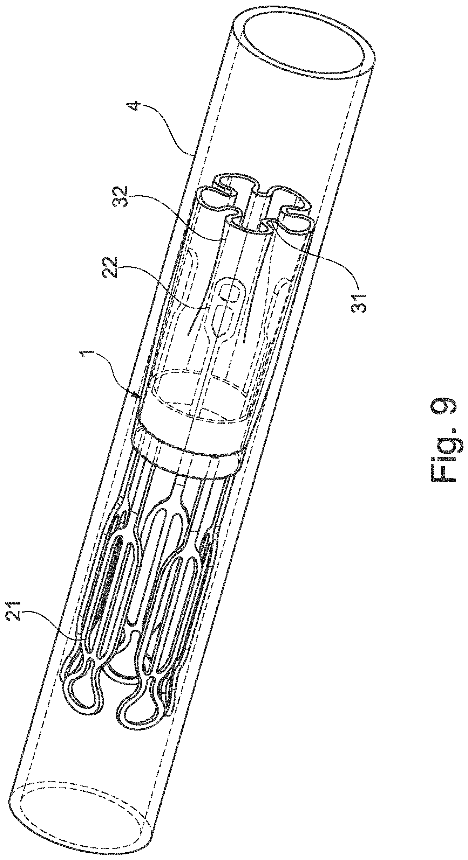

The use of the implant for treatment of pleural effusion is now described by way of example of the embodiment of the implant 1 as shown in Figure 1 and with reference to Figure 9.

For implanting, the clamps 21 may be bent from the original shape of the implant 1 in proximal direction until the clamps 21 align with central ring 20. Also the struts 22 may be bent in distal direction from the original shape of the implant 1 until the struts 22 align with the central ring 20. In this condition, the implant 1 can be inserted into a sheath 5, which can also be referred to as a delivery pipe. This condition of the implant 1, which can also be referred to as the delivery condition or delivery shape, is schematically shown in Figure 9. The sheath 4 can be introduced into the body of the patient from the thoracic direction. Once the sheath 4 passes through an incision in the diaphragm, which has be prepared beforehand, the implant 1 may be pushed out of the sheath 4. When leaving the sheath 4, the struts 22 of the implant 1 together with the valves 31 provided between adjacent struts 22 can expand into their final position which is shown in Figure 1. In this configuration the implant 1 may then be pulled in the proximal direction until the struts 22 and valves 31 rest against the abdominal side of the diaphragm. At that stage, the implant 1 may be pushed out of the sheath 4 further and the clamps 21 can expand to their final position. In the implanted state of the implant 1, the diaphragm is clamped between the clamps 21 and struts 22 and is held in position.

Fluid which is produced in the pleural cavity can enter the implant 1 from the proximal side and is collected in the collecting chamber 4 of the implant 1. Once the pressure in the pleural cavity increases to an amount which is higher by a predetermined value than the pressure in the abdominal cavity, the fluid from the collecting chamber 4 enters the valves 31 via the inlet opening 311 and can be released from the valve 31 via the outlet opening 310 of the valve 31 and - if provided - also via the additional outlet opening 312 of the valve 31. This flow of fluid through the valve 31 is schematically shown in Figure 8a.

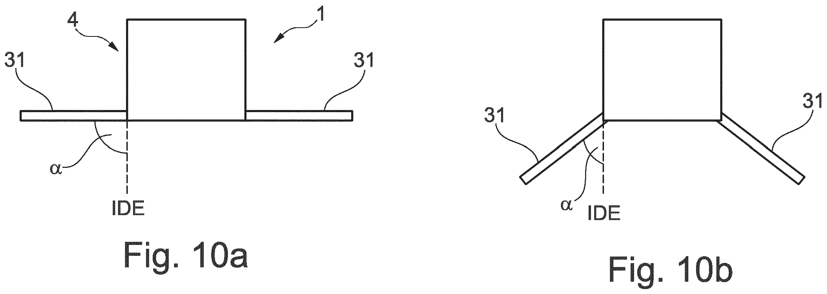

In Figures 10a and 10b schematic cross-sectional views of different embodiments of the valves 31 of the implant 1 are shown. For better visibility, the clamping arrangement is not shown in these figures. The valves 31 are shown in the position they will assume in the final shape of the implant after implantation.

In Figure 10 a, the valves 31 extend from the wall of the collecting chamber 4 in a perpendicular direction and extend radially outward from the wall of the collecting chamber 4. In this embodiment, the angle α between an imaginary distal elongation (IDE) of the wall of the collecting chamber 4 and the distal side of the valve 31 is 90°. In Figure 10b the valves 31 extend radially outward from the wall of the collecting chamber 4 and at angle α between an imaginary distal elongation (IDE) of the wall of the collecting chamber 4 and the distal side of the valve 31 of less than 90°.

The valves 31, for example, can be held in the depicted positions of Figures 10a and 10b by respectively shaped struts (not shown).

As far as applicable, individual features, which are present in the described embodiments can be combined with each other or with other features or can be exchanged, without leaving the scope of the invention.

The implant can serve as a shunt from the thoracic cavity, in particular pleural cavity, to the abdominal cavity. The manufacturing of the implant and its handling during implanting are facilitated. The implant can be provided by video-assisted thoracoscopic surgery (VATS). The implant can be placed in the non-muscular part of the diaphragm, whereby the impact on the patient can be further reduced.

### Reference Numerals

- 1: implant
- 2: core body
- 20: central ring
- 200: proximal end
- 201: distal end
- 21: clamp (proximal arm)
- 210: radial end
- 211: hole
- 212: bending section
- 22: strut (distal arm)
- 220: radial end
- 221: hole
- 222: bending section
- 3: cover
- 30: collecting portion
- 31: valve
- 310: outlet opening
- 311: inlet opening
- 312: additional outlet opening
- 313: sealing line
- 314: connecting stripe
- 32: section for strut
- 33: central portion
- 4: collecting chamber
- 5: sheath
- R1: bending radius
- R2: bending radius
- D1: inner diameter central ring
- D2: outer diameter central ring
- D3: diameter struts
- D4: diameter implant
- H1: maximal height for receiving tissue
- H2: height central ring
- H3: height implant
- F: Fluid
- DIST: distal direction
- PROX: proximal direction
- IDE: imaginary distal elongation
- α: angle between IDE and valve

## Claims

1. Implant for treatment of pleural effusion **characterized in that** it comprises a collecting chamber (4) with a proximal inlet, at least one distal lateral valve (31), which is connected to the collecting chamber (4) for draining fluid from the collecting chamber (4), and a clamping arrangement for attaching the implant (1) to tissue.

2. Implant according to claim 1, wherein the implant (1) comprises a core body (2) comprising a central ring (20) having a distal end (201) and a proximal end (200), wherein the implant (1) further comprises a cover (3) comprising the at least one valve (31) of the implant (1), a collecting portion (30) at least surrounding part of the central ring (20) and a central bottom portion (33), wherein the collecting portion (30) and the central bottom portion (33) form the collecting chamber (4).

3. Implant according to claim 1 or 2, wherein the implant (1) comprises at least two distal lateral valves (31).

4. Implant according to claim 2 or 3, wherein the clamping arrangement comprises at least two proximal arms (21) at least partially extending radially outwards from the central ring (20) and at least two distal arms (22) at least partially extending radially outwards from the central ring (20).

5. Implant according to claim 4, wherein the proximal arms (21) partially extend over the proximal end (200) of central ring (20) in proximal direction.

6. Implant according to anyone of claims 4 or 5, wherein the distal arms (22) are offset to the proximal arms (21) in a circumferential direction of the central ring (20).

7. Implant according to anyone of claims 2 to 6, wherein the core body (2) is formed as a single component.

8. Implant according to anyone of claims 4 to 7, wherein the distal arms (22) comprise a bending section (222), where the distal arms (22) are bent from the distal direction into a radially outward direction, and wherein the bending section (222) extends distally over the distal end (201) of the central ring (20).

9. Implant according to anyone of claims 4 to 8, wherein the proximal arms (21) at least partially extend into a radially outward direction and comprise a bending section (212), where the proximal arms (21) are bent from the proximal direction to a direction between the proximal and distal direction and wherein preferably the radial outer ends of the proximal arms lie in the level of the distal arms (21).

10. Implant according to anyone of claims 2 to 9, wherein the core body (2) is made of metal, in particular a shape memory alloy, preferably Nitinol, and the cover is made of a plastic material, in particular PTFE or silicone or wherein the core body (2) is made of a plastic material, in particular TPE, silicone or rubber.

11. Implant according to anyone of claims 4 to 10, wherein the core body (2) comprises three distal arms (22) and three proximal arms (21), preferably five distal arms (22) and five proximal arms (21).

12. Implant according to anyone of claims 1 to 11 and wherein the at least one valve (31) comprises an outlet opening (310) at its radial outer end and preferably wherein the at least one valve (31) has at least one additional outlet opening (312) at the distal side of the valve.

13. Implant according to anyone of claims 2 to 12, wherein the at least one valve (31) is formed in the cover (3) between adjacent distal arms (22) of the core body (2).

14. Implant according to anyone of claims 1 to 13, wherein each of the at least one valves (31) is formed by two plastic layers.

15. Implant according to claim 2 to 14, wherein the cover (3) comprises a distal layer, which comprises the central portion and the distal layer of the at least one valve, and a proximal layer, which comprises at least a collecting portion (30) and the proximal layer of the valves (31).

16. Implant according to anyone of claims 2 to 15, wherein the cover (3) individually encloses each of the distal arms (21) and the proximal arms (22).
